# EUROPEAN PATENT SPECIFICATION

(11) **EP 1 151 748 B1**
(45) Date of publication and mention of the grant of the patent: **27.10.2004**
(21) Application number: 01201558.2
(22) Date of filing: 01.05.2001
(51) Int. Cl.: A61K 9/50

(54) **Continuous manufacturing procedure of microcapsules for extended release of water soluble peptides**
Kontinuierlicher Prozess für die Herstellung von Mikrokapseln mit verzögerten Freisetzung von wasserlöslichen Peptiden
Procédé de préparation contenant des microcapsules à libération prolongée de peptides hydrosolubles

(30) Priority: 03.05.2000 AR 0102102
(43) Date of publication of application: 07.11.2001
(73) Proprietor: Eriochem, S.A., Parana, Entre Rios (AR)
(72) Inventor: Moyano, Nora, Parana-Entre Rios (AR); Iturraspe, Jose, Parana-Entre Rios (AR); Nunez, Jose Lucio, Parana-Entre Rios (AR)
(74) Representative: SUGRANES - VERDONCES - FERREGÜELA

(56) References cited:
- WO-A-00/72955
- WO-A-95/13799
- WO-A-98/35654
- CH-A- 653 553
- US-A- 5 476 663
- US-A- 5 733 567
- YUH-FUN M ET AL: "Liquid-liquid emulsification by rotor/stator homogenization" JOURNAL OF CONTROLLED RELEASE, ELSEVIER SCIENCE PUBLISHERS B.V. AMSTERDAM, NL, vol. 38, no. 2, 1 February 1996 (1996-02-01), pages 219-228, XP004037407 ISSN: 0168-3659

## Description

### Invention field:

The invention is related to a pharmacotechnical continuous method for the manufacturing of microcapsules, constituted by biodegradable and biocompatible polymeric material that contains an active peptide by complex emulsion formation, water/oil/water type (W/O/W). The procedure has been developed in order to obtain this microcapsules in sterile injection form, that allow a controlled administration into adjustable release periods between 1 and 18 weeks, of some soluble or dispersible in water drugs, used for the treatment of neoplastic and gynaecological diseases, and other problems.

In this way, the invention includes pharmacology field, and particularly, a specific pharmacotechnical way for the manufacturing of injectable, controlled release medicines.

### State of the art:

Since the first work about encapsulation by coacervation invented by B.K. Green for the NCR (Patent US2800457, 1957) directed to the development of copy papers, a number of publications and books, related to microencapsulation of natural or synthetic substances into polymeric walls, have been written, in order to apply them in prolonged or controlled release uses of that substances (Microcapsule Processing and Technology, Asaji Kondo, 1979, Marcel Dekker). The gradual release of substances in controlled time intervals is important in pharmaceuticals drugs, foods, agrochemicals, fertilizing, and other fields. A notable development, according to the number of publications observed in the recent years, corresponds to the subject of microencapsulation of active pharmaceutical ingredients (Microspheres and Drug Therapy, Ed. Stanley S. Davis and others, 1984, Elsevier; Controlled Release Systems: Fabrication Technology, Vol I and II, Ed. Dean Hsieh, 1988, CRC Press, Inc.; Polymeric Drugs and Drugs Delivery Systems, Ed. Richard L. Dunn, 1991, ACS Symposium Series 469; Microencapsulation of Drugs, T.L. Whateley, 1992, Harwood and "Sustained Release Injectable Products", Ed. J. Senior and M. Radomsky. Interpharm Press, Denver, Colorado, USA, 2000). This complex physicochemical process has become a specialty.

Referring to the pharmaceutical substances field, and as a result of clinical studies made by drug administration specialists, it has been determined in many cases that better therapeutic or pharmacological effects can be obtained, by means of the use of procedures of continuous infusion of the drug, that when the same is administered by conventional methods, in injectable, oral form, or by other ways. In these cases, it is necessary to consider the use of prolonged release technology of active ingredients, which also include injection, oral, and others like subcutaneous implants.
Generally, the substitution of a conventional method by one of slow release produces smaller collateral effects, correlated with drug concentration peaks in the organism, when the minimal required active concentration is exceeded. One of those prolonged release systems is the microcapsules of polymers containing active agents like polypeptides, proteins, hormones, nucleotides, and chemotherapy drugs, among others. Once the microcapsules are administered to the organism, drugs may be released by diffusion through a semi permeable wall in some cases; by wall dissolution in others; or by multiple mechanisms that include mainly the biodegradation, of encapsulating polymer into the living tissues, to biocompatible fractions that follows a metabolic route for absorption or elimination.

This polymer biodegradation process produces, therefore, the slow dosage of active ingredient.

Microcapsules based on re-absorbable and/or biodegradable polymers or co-polymers, have been the object of extended investigation, related to manufacturing materials and methods; so as administration routes. Actually, an increasing application for administration of biotechnology products is being found, including water soluble, slight soluble, and not water soluble substances. There are several administration routes for this particular type of microcapsules, depending on the drug to be released. They can be adapted to injectable; as well as, for the administration to the gastrointestinal system, nasal mucous, and other access routes.

With the condition that they are degraded into biocompatible residues, a great number of polymers with a main hydrophobic chain may be used to form the microcapsules wall, and occasionally require a special purification grade. Among the biodegradable polymers generally used we can mention poly(d,l-lactic) acid; poly(d,l-lacticglycolic) copolymer; poly (caprolactones); poly(hydroxybutirate); poly(orthoesters); poly(anhydrous), so as the mixture of them and other polymers ("Polymeric Drugs and Drug Delivery Systems", Ed. Richard L. Dunn, 1991, ACS Symposium Series 469, p. 15-20).

Poly(d,l-lactic-glycolic) acid, d,l-lactic acid and glycolic acid copolymer, abbreviated as PLGA, and the homopolymer of d,l-lactic acid, poly(d,l-lactic) acid, abbreviated as PLA, have been used since 1973 as polymers for medicine microcapsules. Among several examples, we can mention the microencapsulation of a narcotic antagonist like naltrexone (J. H. R. Woodlnad et al., J. Med. Chem., vol 16, 897, (1973); S. E. Harrigan et al., Midl. Macromol. Monogr., vol 5 (Polym. Delivery Systems), vol 91 (1978)); of anaesthetic substances (N. Wakiyama et al., Chem. Pharm. Bull., vol 30, 3719, (1982)), and of steroids (D. L. Wise et al., J. Pharm. Pharmacol., vol 32, 399, (1980)). We can specially mention the use of PLGA 50:50 and 69:31 (molar ratio of lactic acid:glycolic acid) in nafarelin acetate microencapsulation, a luteinizing hormone release hormone analog (LH-RH), 200 times more powerful than LH-RH (L. M. Sanders et al., J. Pharm. Sci., vol 73, 1294-1297, (1984)). Actually, is completely accepted the use of PLGA and PLA as biocompatible polymers and degradable to toxically accepted products, that are eventually eliminated from the body (D. H. Lewis, Biodegradable Polymers as Drug Delivery Systems, Ed. M. Chasin et al., Marcel Dekker, New York, NY, pp 1-42, 1990).

To obtain PLA or PLGA of controlled molecular weight, a polycondensation reaction is carried out on cyclic dimers from lactic acid and glycolic acid, known as lactide and glycolide. There is an extensive literature about synthesis and purification methods of PLA and PLGA with molecular weights ranging 20000 Dalton or less. Among the direct polycondensation procedures it can be mentioned, those that are made without catalyst, those that use metallic catalyst, described in several patents, like the following ones: US3297033 (1967); US3773919 (1973), and US3839297 (1975); and those that use acid catalyst like ionic exchange resins proposed in patent US4273920 (1981).

Slow release microcapsules as administration forms of hormones, antibiotics, anti-inflammatory substances, antitumoral drugs, antihypertensive drugs, antipyretics, vasodilators, antiallergic agents, and analgesics, where PLGA o PLA is the constitutive biodegradable wall material, are particularly known.

For the purpose of the present invention, are particularly interesting the microcapsules containing biologically active substances, that are water soluble or can be able to form a suspension into an aqueous phase, and in which this aqueous phase is contained, principally dispersed into a rigid portion of biodegradable polymer. Among the water soluble drugs, active peptides and specially hormones, are indicated. A specially interesting water soluble hormone, is leuprolide acetate, which was synthesized almost simultaneously by J. A. Vilchez-Martinez et al., (Biochem. Biophys. Res. Commun. 59, 1226, (1974)) and by Fujino et al. (M. Fujino et al., Biochem. Biophys. Res. Commun. 60, 406-413, (1974)), and it is the first superactive agonist of luteinizing hormone release hormone (LH-RH), with approximately 10 times the biological activity of LH-RH. It has been used for the treatment of hormone dependent tumors, such as prostate cancer (T. W. Redding et al., Proc. Nat. Acad. Sci. USA, vol 78, 6509-6512, (1981)) and breast cancer (E. S. Johnson et al., Science, vol. 194, 329-330, (1976)); of endometriosis (D. R. Meldrum et al. J. Clin. Endocrinol. Metab., vol. 54, 1081-1083, (1982)); of uterine fibrosis (M. Filicori et al., Am. J. Obstet. Gynecol., vol. 152, 726-727, (1985)).

During studies done by H. Okada et al., about vaginal absorption of leuprolide in rats, it was observed that constant levels of drug in blood produce higher castration than its intermittent and pulsating administration, and it was thought that a slow release injection should produce optimal therapeutic results (H. Okada et al., J. Pharm. Dyn., vol. 6, 512-522, (1983)). This originated the development of the named depot injection, suitable until 120 days of leuprolide acetate release (H. Okada et al. Jap. Patent Appl. 207760 of 1983, which corresponds to US4652441 (1987); Y. Ogawa et al. Chem. Pharm. Bull., vol 36, 1095, (1988)). Other hormones of particular interest for the present invention, agonists of the luteinizing hormone releasing hormone (LH-RH), are the goserelin acetate (US4100274), the buserelin acetate (US4024248), the triptorelin acetate (US4010125) and the nafarelin acetate (US4234571)

A number of methods have been developed until present for the microencapsulation of active ingredients into biodegradable and not biodegradable polymers. Among these, three main types predominate: those of emulsion/separation of phases; those by "spray" drying encapsulation; and those based on solvent evaporation into an aqueous or organic vehicle phase.

In emulsion/separation of phase techniques, an aqueous solution of the drug, or in powder state drug, is dispersed into an organic solution containing the polymer. Once the emulsion is formed, a coacervation agent is added, generally a vegetal or mineral oil, which induces the microspheres formation, containing the active ingredient. As examples, patents US4675189 (1987) and US4835139 (1989) can be mentioned. These methods have the disadvantage of using great amounts of solvents and oils. The microcapsules formation stage also depends on polymer quantities, solvent, and coacervation agent. An additional undesirable effect is the tendency of particles to adhere each other during manufacturing process.

Encapsulation methods, by "spray" type drying, consist in initially preparing an aqueous phase, which contains the active agent in solution or suspension. This medium is dispersed into an organic phase that contains the polymer to give a water/oil (W/O) type emulsion, which is pulverized in a hot air flow, into a drying equipment. Microcapsules are formed by organic solvent evaporation. One version for semi-continuous manufacturing of peptide microcapsules, including leuprolide acetate, is presented in patent US5622657 (1997), where the water/oil emulsion is conducted to microspheres formation, by "spray" type drying with simultaneous spraying from an auxiliary nozzle of an aqueous solution containing a substance that contributes to avoid the adherence between particles during its formation.

Procedures that include solvents evaporation from an aqueous or organic phase, are the most common for manufacturing of microcapsules. Basic technique consists in dispersing the drug in a polymer solution of organic solvent. The active ingredient may be a suspended powder or it be dissolved into an emulsionable solvent in the polymer solution. This first dispersion is emulsioned in a solvent , which is called vehicle, non miscible with the solvent of polymer, which is then evaporated.

There are a variety of techniques based on solvents evaporation, developed for the microencapsulation of water soluble and non water soluble substances.

American patent US3691090 (1972) describes the encapsulation of water soluble substances, including medicines, where it is proposed to disperse the substance into an organic solvent, which is miscible or partially miscible in water. The polymer is dissolved in that solvent and the organic phase is emulsioned into an aqueous medium containing an inorganic salt to avoid the organic solvent solubilization. The resulting type oil/water (O/W) emulsion contains oily microspheres of polymer containing active substance. Microcapsules are consolidated by organic solvent evaporation.
In patent US3960757 (1976), encapsulation of insoluble, or slightly soluble medicines is proposed by the technique of dissolving or dispersing the active substance into a polymer solution of an organic solvent which is almost insoluble in water. The organic solvent must have a vapor pressure greater than the water one. Organic phase is emulsioned in a vehicle consisting in an aqueous solution of a hydrophilic colloid or a surfactant agent, producing an oil/water (O/W) biphasic system. The process follows with the removal of the organic solvent by evaporation, so the microcapsules consolidate. As hydrophilic colloids, gelatin, polyvinyl alcohol (PVA), carboximethylcellulose, and others, are proposed. Among the proposed solvents used to dissolve polymer, some chloroalkanes like dichloromethane, ethylene chloride, chloroform, and others, are mentioned. The usual polymers are the hydrophilic type.

In patent US5540973 (1996) a process is described, to prepare microspheres with LH-RH and its analogs, in a biodegradable and non water soluble polymer matrix, where the process begins dissolving the polymer into a first organic solvent, and then the hormone is dispersed with agitation. Then, this first solvent is evaporated to dryness, and the residual mass is dissolved into a second solvent where the polymer dissolve, but not the active drug, which stays in suspension. The final stage is the preparation of an oil/water (O/W) emulsion, with the aggregate of a surfactant agent, and the evaporation of the second solvent to give rise in this stage to the formation of microspheres.
Of particular interest for the present invention is the procedure of microencapsulation, that uses a drying process in the liquid, or complex emulsion method, as was called by Asaji Kondo ("Microcapsule Processing Technology, 1979, Marcel Dekker, cap 10, p. 106); and more specifically, the in-water drying method, proposed since 1964 in several patents, like: JP39-28744 (1964); JP42-13703 (1967); JP43-10863 (1968) and FR1362933 (1964), consisting in encapsulation of aqueous solutions which initial stage is the formation of an aqueous phase in oil emulsion (W/O), that can be then encapsulated in another major aqueous phase [(W/O)/W], this is, a water in oil emulsion, emulsioned in water, is prepared.

This method has a number of advantages; particularly, it doesn't need pH adjustments, nor an important heat source, nor the use of a special reagent agent, so chemically not stable materials, can be micro encapsulated without substantial degradation. Other advantages exist, that depend on the control that may be done on the physicochemical conditions of the preparation, among them it can be mentioned: better yields of the microcapsules free from agglomeration, and better efficiency in active ingredient encapsulation, compared with the other described methods. It can also be mentioned the possibility of using this process to prepare 0.25 to 1.0 g of active ingredient batches, considering cases in which this is very expensive, also making easy the process scale-up to amounts rising 10 and 100 g of active ingredient.

Essentially, microencapsulation by in-water drying of complex emulsion method, consists in the preparation of the first water in oil type emulsion (W/O) by dispersing the active material in aqueous solution of a volume V, into an eight times V volume of a solvent partially or totally immiscible in water, where the polymer that will form the microcapsule wall was dissolved. This solvent must have a lower boiling point, and a vapor pressure greater than water, so it can be evaporated in presence of water. Separately an aqueous solution is prepared, that contains a stabilizer, a protective colloid, with a 40 V volume, and microencapsulation is made by agitation of the last solution, while the dispersion (W/O) is added, to obtain a total volume near to 50 times V, of a water in oil in water double emulsion [(W/O)/W]. This system is stable, and the fluid state microcapsules, made of an organic solution of the polymer that possesses dispersed into its interior micro- and nano-drops of an aqueous solution of active ingredient, are emulsioned in the external aqueous phase. When the organic solution polymer is dried by heating and/or reduced pressure, the polymeric matrix that forms the microcapsule becomes hard, and the aqueous micro-drops or nano-drops of active ingredient remains contained into the microcapsule.

Size and stability of the Microcapsules is mainly influenced by factors like emulsion viscosity (W/O), local agitation intensity, temperature, and the aggregate of some additive substances in the aqueous phases. Using this method, 1 micron to several hundred of microns microcapsules may be prepared. In the first emulsion preparation (W/O), it is convenient, in some applications, to aggregate hydrophilic substances dissolved in water, which act like retention agent of the active substances and include, among others, albumin and gelatin (FR1362933 (1964); JP43-10863 (1968)). These substances contribute to stabilize emulsion (W/O) to avoid the coalescence of micro-drops. On the other hand, in the second emulsion preparation [(W/O)/W] it is recommended to dissolve previously, a hydrophilic protective colloids that work as stabilizers in the external aqueous phase, among which gelatin and polyvinyl alcohol are mentioned. (PVA) (FR1362933 (1964); JP42-13703 (1067); A. Kondo, Ind. Chem. (Japan), 72 (2), 493 (1969)). These colloids must also be slightly soluble in the organic solvent, where the first emulsion is produced (W/O). If no protective colloid is used, the active agent entrapment in the microcapsules is notably reduced, and an inversion of microcapsule may be produced, a particular situation where the aqueous internal core is released to the external aqueous medium, and only polymer microspheres are formed. Even though the results of the process are strongly depending to selection and particular molecular properties, so the hydrophilic protective colloid as from the active ingredient retention substance of the first emulsion, in the literature, as much from patents as from scientific publications, a number of possible substances are mentioned without giving so many specifications about them.

A disadvantage of in-water drying is that it takes a long time to eliminate the solvent from the polymer solution, which includes the micro-drops containing active ingredient. If the solvent is very rapidly eliminated, little orifices and bubbles are formed on the surface of microcapsules. One way to diminish these problems is to extract the organic solvent with another solvent which is water miscible and soluble in the organic solvent, but which may not dissolve the polymer (Gevaert, Photo-Production N. V., FR1362934 from 1964). Another way to proceed is making a controlled evaporation of the solvent, by a gradual heating combined with a pressure reduction.

In the complex emulsion in-water drying, it is preferable that the organic solvent and the polymer were not miscible with the active ingredient, so it could be encapsulated. It may be in an aqueous solution or dispersion, or as a solid powder. In an aqueous solution, if the dissolved active drug has a low molecular weight, it will have the tendency to diffuse through the microcapsule wall, during encapsulation process. On the other hand, if it is a molecular substance with a molecular weight of several thousands of Dalton, it will be retained inside the microcapsule.

The use of this complex emulsion in-water drying method, for encapsulation of highly hydrophilic pharmaceutical drugs is frequent in the state of the art. Discontinuous procedures are described, to obtain prolonged release microcapsules for injectable use, for implants, transdermal, or for oral administration. Among other patents, EP0765659, US4652441, US4954298, US5271945, US5330767, US5611971, and US5651990 can be mentioned.

Discontinuous encapsulation procedures of water soluble peptides, for pharmaceutical use, using this complex emulsion in-water drying method, and the use of PLGA and PLA as encapsulating polymers, show some difficulties like a high dispersion of particles sizes, ranging from 1 to more than 400 microns, micro particles adhesion , process regulation difficulties, and poor reproducibility.

In the case of the first emulsion (W/O), the accomplishment of a process in discontinuous form implies intensities of agitation and variable times of mixture, not only based on the size of process, but also based on other variables, which may include the recipient size and shape, since, due to the high viscosity of the phases, a good agitation or mixing of the total mass cannot be obtained, but the stress caused by anyone of the applied methods (agitation turbines, dispersers, or ultrasound), can only be transmitted to millimeters of the applying point. This determines the high dispersion of particle size in the first emulsion (W/O).

The second emulsion, in which the total external aqueous phase is into one reactor, to which the emulsion (W/O) is added slowly to form the complex emulsion [(W/O)/W], is very dependent of factors like: adding time, temperature, initial volume of first emulsion to second emulsion ratio, polymer concentration in organic phase, kind and concentration of protective colloid in the second aqueous phase, and injection point position of (W/O) emulsion. Consequently, the control of a process of discontinuous type, is extremely complicated, and gives as a result a high dispersion of particle size, and low yields of the micro encapsulated material which pass through a mesh 200 (75 microns), maximum convenient size for injectable preparations. It has been observed that, as a result of discontinuous traditional manufacturing procedures, percentages near to 30% of microcapsules with a diameter greater than 75 microns are obtained.

When the second emulsion is formed, by adding the first emulsion over a total volume of the aqueous phase where the microcapsules will be formed, an important factor to take in mind is the feed place of the first emulsion, because, when external aqueous phase is agitated strongly, different sized particles may be formed practically in the total volume, and, when these particles reach a size in which the reliable superficial evaporation of the volatile solvent allows the hardening of the microcapsule, this one no longer can diminish in its size although it is put under greater times of agitation. This determines a great dispersion in the size of final particles, because the same ones can form in places moved away of the point of applying the stress where the microcapsules reach a great size, whereas in the neighborhoods of the applying point of the stress, the microcapsules of minor size will form.

Also regarding known methods for the manufacturing of microcapsules, other documents dealing with the problems of microcapsules formation must be mentioned.

In particular, WO 9513799 (1995) discloses the difficulty of controlling the size of the microcapsules and the problem of scaling-up and maintaining the critical parameters to obtain a uniform population of microcapsules. All theses problems, which are caused by the use of dynamic mixing techniques of the phases (W/O), are resolved in WO 9513799 (1995) using a static mixer to form (W/O) emulsions and to obtain the microcapsules with the desired features.

WO 9835654 (1998) deals with the problem of producing small particles that exhibit all the desired properties of drug incorporation, with low residual solvent and good scalability. It refers to processes for preparing microspheres using multiple phases, obtaining small microcapsules by: a) introducing a continuous phase and a dispersed phase with an active agent into a reactor; b) continuously transferring the emulsion to a solvent removal vessel to obtain a population of an average of particle size; c) and to produce a second larger population of particles by continuously performing steps a) and b).

Also relating to complex or multiple phases methods to form microcapsules, it is disclosed in US 5,476,663 (1994) a microcapsule for injection, particularly for biologically active polypeptides, which comprises particles containing a water soluble drug. The microcapsule is obtained by mixing continuously a stable oil-in-water emulsion in an aqueous layer to give a W/O/W ternary layer, desorbing further the solvent in the oil layer to give microcapsules. This desorption is achieved by conventional techniques.

In the same way, US 5,733,567 (1995) discloses a process for preparing a pharmaceutical composition in the form of microspheres which comprises the steps of dissolving the active principle in water; emulsifying the aqueous solution in an hydrocarbon matrix; emulsifying the first emulsion with an external aqueous phase with a surfactant; and extracting the solvent.

Finally, another way to obtain improved microcapsules with a reduced loss of the active ingredient is the method disclosed in WO 0072955 (2000), wherein micro and nanoparticles are obtained, consisting of natural substances and/or substances of biological synthesis, by means of a micromixer with a mixing camera with microcanals. This method permits the obtention of microcapsules with no agglomeration, without any toxicological substance and organic medium and without any problem for scaling up the same.

As it was mentioned previously, microcapsules consolidate when the superficial evaporation of the volatile solvent used to dissolve the polymer, determines the surface hardening, so that no longer the subdivision of microcapsules of minor size is possible. This evaporation is very dependent on the capacity of the water to eliminate de organic solvent by absorption, which in the case of methylene chloride reaches a solubility of about 1.3% in weight at room temperature. In a discontinuous system this absorption capacity is variable with time, since, at the beginning of the operation the microcapsules are formed into a medium where only there is water with a protective colloid with tensoactive power, whereas at the end, a complex water-tensoactive system, and increasing amounts of solvent and microcapsules, are obtained, situation in which it increases the probability of particles agglomeration.

Several procedures described in the literature include, after the separation of external aqueous phase, water wash and drying to eliminate moisture, milling, and sieving of the dried product, to eliminate particles agglomerates and homogenize its granulometry, and finally, the dosage as a solid to obtain a final product. These operations with solids present the difficulties and require the characteristic cares of operations with injectable pharmaceutical powders. Expensive equipment may be disposable to ensure sterility conditions, no contamination and no moisturizing of microcapsules, since the material is extremely hydrophilic, presents a high specific surface and must contain not more than 1% of water.

On the other hand, in discontinuous methods, active peptide losses, until of 70% have been registered throughout all the process. These losses are evaluated comparing the active peptide amount used as active principle, and the one that is retained in the microcapsules of usable sizes as injectables. The losses results from adding the active peptide that is entrapped in microcapsules greater than 75 microns, plus the peptide that is dissolved in the not emulsioned medium, plus the drug that goes away with the washings with distilled water, plus that which is entrapped in very small microcapsules that also go away with the washing.

### Memory:

Due the difficulties that the methods for manufacturing microcapsules of pharmaceutical grade presents when using the complex emulsion type W/O/W method, and PLGA and PLA as encapsulating polymers, ERIOCHEM SA has developed a complete continuous method the manufacturing of microspheres, which uses two intensive agitation equipments in cascade to obtain the complex emulsion, following the process as liquid suspension until the fractionating, freezing under agitation and lyophilizing the finished product into their final package. It is managed in this way to reduce the steps of the process; to improve the repetitivity of the process variables, facilitating consequently the control; to obtain a narrow and reproducible size particle distribution, composition and internal distribution of the drug, which allows to obtain microcapsules with controlled release periods of the active drug; to ensure a high retention grade of drug inside the microcapsules, which minimizes the lose of high cost active peptides; substantially improve the yield of particles with the desired size; to minimize the product exposition during the process steps, which diminishes sensibly the costs on equipment inversion to ensure sterility condition, and low contamination with particles, considering that the main uses of microcapsules are injectable forms; improve lyophilized quality referring to drying homogeneity and resuspension facility due to the freezing system with agitation, which renders a homogeneous suspension.

These advantages of the new procedure, consequently, improve the productivity of the actually known procedures, and also improve the final product quality.

This procedure, the object of the present invention, that is used for prolonged release microcapsules of water soluble peptides, for injectable forms and with a adjustable release between 1 and 18 weeks, begins emulsionning continuously, an active peptide in aqueous solution containing a retention substance, in an oily solution of a biodegradable polymer dissolved in an organic solvent which is very slight soluble in water, using for that intention a first equipment of intensive agitation, closed to ambient, fed by dosage machines. This first water/oil emulsion, after being cooled, is fed by a dosage machine to a second equipment of intensive agitation closed to ambient, where it is emulsioned in continuous way in a vehicle aqueous phase containing an hydrophilic protector colloid, which is fed by a continuous dosage machine; this second complex emulsion type water/oil/water is taken in continuous way to a closed recipient, where the solvent is evaporated by pressure reduction, producing the microcapsules consolidation; then this microcapsules are put under the following operations in wet way: they are sieved in suspension; then they are centrifuged; they are washed with water; and they are fractionated into adequate recipients, dispersed into an aqueous medium containing a lyophilizing excipient; following they are frozen in an orbital agitation freezer until temperatures below -20°C, and they are lyophilized into the same recipients.

These microcapsules may be used for water soluble peptides administration, like implants or orally.

The advantages and characteristics of the present invention are better explained through the following detailed description, in which a numeric reference is made to each detail that can be observed in the attached drawings, that along with the accomplishment examples constitute a preferred procedure, without for that reason establishing restrictions with respect to the invention, individually referring to used details, materials and equipment, being:

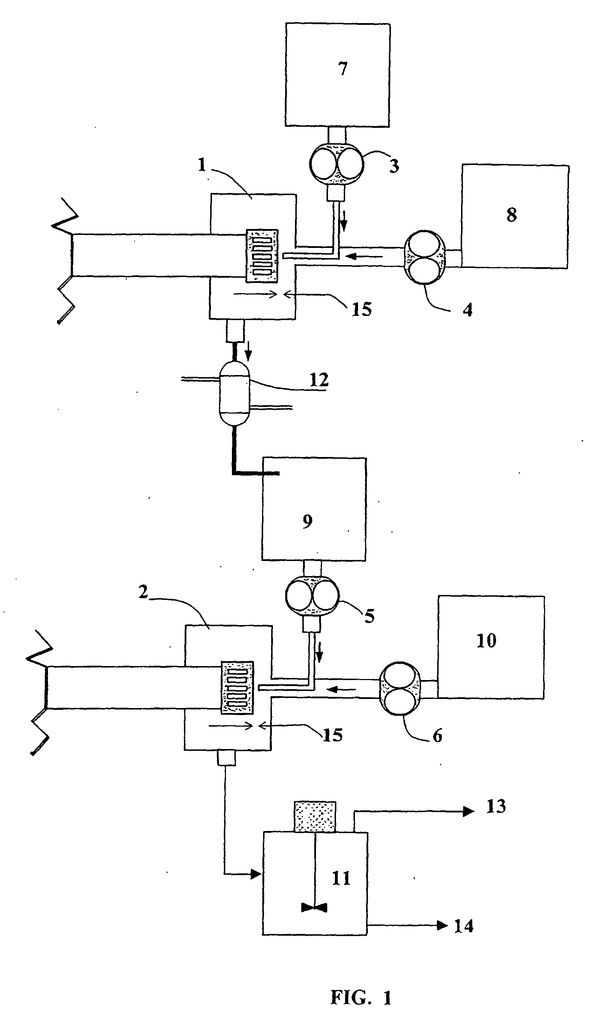

Figure 1 represents a scheme of the first stages of the continuous process for microcapsules manufacturing
   References:
   1: First intensive agitation equipment. In the draw it is represented a rotor-stator assembly.
   2: Second intensive agitation equipment.
   3: Active peptide aqueous solution dosage machine.
   4: dosage machine of oily solution of biodegradable polymer in organic solvent.
   5: dosage machine of first water/oil emulsion.
   6: dosage machine of aqueous solution containing protective hydrophilic colloid.
   7: Container of active peptide aqueous solution.
   8: Container of polymer biodegradable in organic solvent oily solution.
   9: Container for cooling first emulsion.
   10: Container of aqueous solution containing protective hydrophilic colloid.
   11: Vacuum evaporator with agitator.
   12: Refrigerator on line.
   13: Vacuum line.
   14: Finished microcapsules line that follows to centrifugation, washing process, fractionating, package, agitation freezing, and lyophilization.
   15: Distance between the inside feed tube in the intensive agitation equipments and the agitating element, which must not be greater than 20 mm.

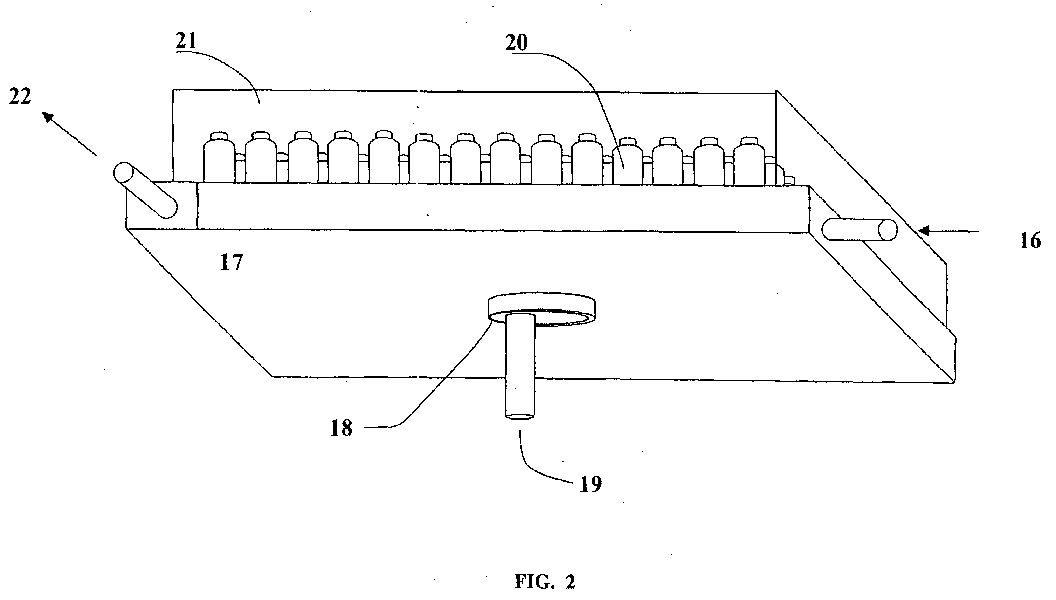

Figure 2 represents a scheme of orbital agitation freezing equipment
   References:
   16: Entrance of refrigerating liquid.
   17: Refrigerating plate where the refrigerating liquid circulates at very low temperatures.
   18: Eccentric system that allows circular movement of the plate.
   19: Eccentric axis that ties to electrical motor of adjustable speed.
   20: Final packages containing the suspension of microcapsules already fractionated.
   21: Plate covering that avoids the ambient humidity condensation, and keeps the recipients in a closed ambient.
   22: Exit of refrigerating liquid.

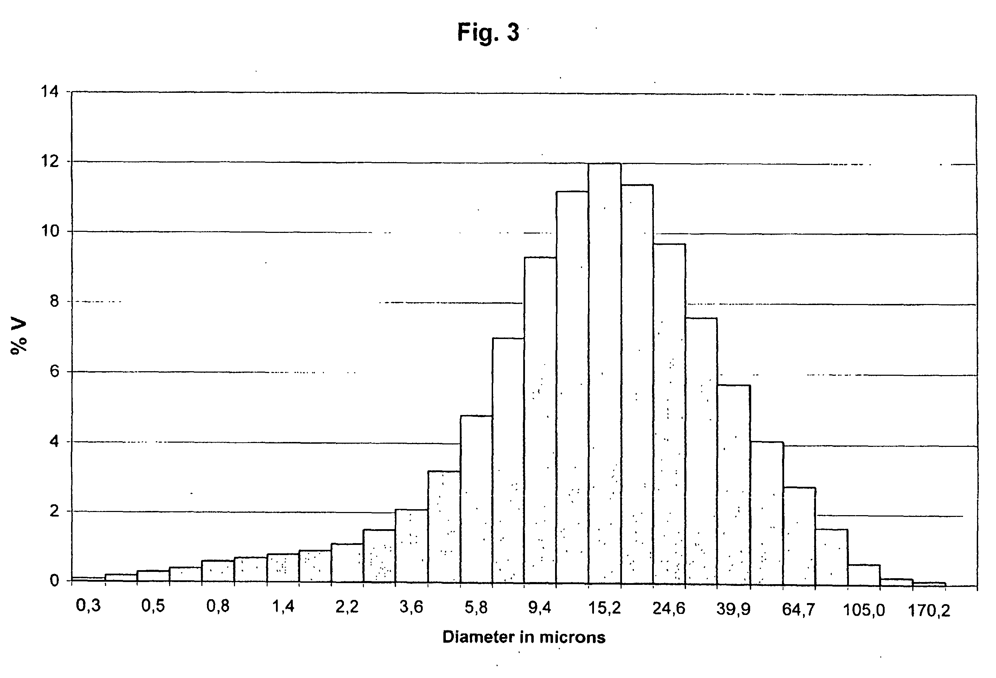

Figure 3 is just a graphic that represents the diameter dispersion of microcapsules obtained in example number 1. It can be seen the volumetric percentage of particles with diameter indicated in the diameters axis (in microns). The diameter distribution analysis of particles by laser interferometry was made. A profile of average diameter of around 15 microns, and one fraction less than 2.5% of greater than 75 microns diameters, is observed.
   Microcapsules resulting from examples 1 and 2, were put under in vivo tests where release kinetic of leuprolide acetate was evaluated in Wistar rats. The obtained results are shown in figure number 4. Horizontal axis shows the days since inoculation, while the vertical axis indicates leuprolide acetate concentration, measured in nanograms per milliliter in blood. The leuprolide acetate concentration was measured by Radioinmunoassay.
Figure number 5 is just an extension of figure number 4. Next, the steps of procedure and the details of process necessary carry out the present invention, are described with precision:

The substances object of microencapsulation by the procedure of the present invention, are active peptides containing between 5 and 20 aminoacids, that also have the property of being water soluble. As a representative molecules of these active peptides we mention leuprolide, goserelin, nafarelina, triptorelin and buserelin acetates. The dosage forms of this molecule indicate the convenience of administering to patient for periods between 1 and 18 weeks.

To produce the microencapsulation of these substances by the proposed method of the present invention, it begins with the preparation of an aqueous solution of one of the named active peptides in concentrations that can range from 5% to 60% in weight, or more preferentially between 10% and 40% in weight of the aqueous phase. Depending on the peptide to be encapsulated, in this aqueous solution an active peptide retention substance can be dissolved, which concentration is adopted in a range from 0% and 10% in weight, and more preferentially, between 0% and 7,5%. This active peptide retention substance must also have the property of giving a semisolid consistence to the aqueous phase, by possible external actions like refrigeration. Among retention substances that can adjust active peptide release, after intensive laboratory tests gelatin of 70 to 100 Blooms, type B, of bovine origin, is used. The indicated gelatin gives a good response in the function of retaining the active peptide inside the aqueous phase, without hardening excessively this phase. It has been verified that gelatins with other characteristics make the emulsion process to be poor in the final retention of active peptide in the microcapsules. In some cases, depending on the active peptide and the desired release period, it may not be necessary to incorporate a retention substance. This aqueous solution is prepared and it is taken to a temperature between 40 and 65°C to ensure the dissolution.

At the same time, a solution of a biodegradable and biocompatible polymer must be prepared, into an organic solvent that has a very low, or almost no mutual solubility with water. For the present invention objectives, as the maximum solubility limit of the organic solvent in water, a value not greater than 6% in weight on water base is taken. The polymer must also be very slight soluble or insoluble in water. This dissolution allows to obtain an homogeneous aqueous phase of the polymer into the organic solvent. As a result from the process of the present invention, the polymer is finally the constituent of the microcapsules matrix.

Without discarding the mentioned polymers in the state of the art, among the polymers that can be possible to use in the process of the present invention, the homopolymer of (d,l)-lactic acid (PLA) and the co-polymers of (d,l)-lactic and glycolic acids (PLGA) are adopted preferentially, which are soluble in chloroalkanes like methylene chloride; dichloroethane; chloroform and carbon tetrachloride, or ethyl ether; benzene; methyl acetate; ethyl acetate and the mixture of them. To this mixtures also can be added alkanes of low molecular weight. Methylene chloride is used as the preferred organic solvent in the present invention, for polymers PLA and PLGA, because of its good dissolvent power, easy evaporation in the presence of an aqueous phase due to its high vapor pressure, and bactericide action that facilitates the aseptic pharmaceutical process, allowing the sterilization of the used polymer by a chemical agent. Other polymer sterilization methods like ionizing radiation, wet heat, dry heat or filtration through a 0.2 microns membrane, are not recommendable in this application.

The molecular weight of the polymer has influence over some product characteristics like: release speed, temporal profile of biodegradation and distribution of particles size in the manufacturing process. High values of molecular weight are associated with a higher viscosity, and this, with the greater particles formation and a higher peptide release period.

The average molecular weight of biodegradable polymer is selected, in concordance with the purpose of this invention, in a range preferentially between 10000 and 30000 Daltons, being the most desirable range between 12000 and 25000 Daltons.

A specially appropriate polymer, to produce microcapsules with a higher release period, is the polylactic acid (PLA) with a molecular weight between 10000 and 25000 Daltons.

If a copolymer of (d,l)-lactic and glycolic acids is used, or PLGA, the molar relation of the monomers Lactic:Glycolic may be between 100:0 and 50:50.

The polymer concentration into the organic phase is regulated between 10% and 60% in weight; more preferentially between 25% and 45%, being this concentration an important factor in the dispersion grade obtained in the following emulsion of the aqueous phase. Fixing the temperature and the polymer concentration, the viscosity of this organic phase is established.

With the active peptide aqueous solution, and the oily one from the dissolved biodegradable polymer, a mixing and intensive agitation process is made to obtain a first water/oil emulsion. This operation is one of the most critical stages to obtain a particle dispersion of the aqueous phase into the oily phase. For this intention, a continuous procedure is used, which allows to make the emulsion under reproducible and appropriate conditions. As a practical example, proposed in the present invention, the homogenizing device consists in a first intensive agitation, closed to ambient, cylindrical, and which uses an assembly rotor-stator as agitating element. This closed chamber, has as a propose to obtain an agitation intensity enough to reach the required dispersion grade of the aqueous phase, containing the active peptide, in the oily phase.

Respect to the fluid dynamic conditions to produce and maintain this first water/oil emulsion, it has been established that with a 17,5 millimeters of diameter rotor, rotating against a fixed stator with striaes in the assembly rotor-stator, the rotor speed must be between 5000 and 12000 rpm for a total flow of the two phases, included between 30 and 500 ml per minute, thus obtaining a good grade of dispersion and an assured stability of the emulsion for the following operation of second emulsion formation. Moreover, the rotor speed is controlled independently from the other process variables, which allows a regulation adjusted to the desired process conditions. With the obtained results in the homogeniser type device, it is established that this first water/oil emulsion must be made in residence time less than 7 seconds and with the rotor peripheral speeds between 3 m/sec and 12 m/sec. The residence time means the time that phases stay in the intensive agitation equipments, anyone being the type of agitation element.

The scale of this first water/oil emulsion may be diminished or increased changing the agitation chamber and the assembly rotor-stator, until the desired dispersion grade is obtained.

When the experiences take place in laboratory scale or pilot plant scale, this first water/oil emulsion may be done using as a homogeniser device a closed agitation equipment carrying on a sonotrode, which will produce the emulsion by ultrasound. This equipment can also be scaled to a higher production size.

As an alternative version for productions at minor scale, it is possible to make the first water/oil emulsion into an agitation equipment with continuous flow of the aqueous and oily phases, containing as the agitation element a sonotrode, working at frequencies between 20000 and 50000 Hertz; and a power of not less than 30 watt.

Another control variable in the aqueous phase dispensability into this first emulsion is the mass ratio of phases that enter to the intensive agitation equipment. As a result from the experiments made, it can be concluded that is convenient to work with a oily phase/aqueous phase ratio between 3 and 20, more preferentially between 6 and 10. For the aqueous phase dispensability regulation in this first emulsion it is also important to maintain the intensive agitation equipment temperature in values ranging from 10°C to 35°C.

In the process of this invention, the water/oil emulsion produced in the first intensive agitation equipment, is continuously refrigerated by a conventional heating exchanger, and is conducted to a closed intermediate container. The refrigeration is made to stabilize this first dispersion, to induce the aqueous phase jellification, causing the elevation of its viscosity. The temperature values that this first emulsion must reach are between 5°C and 25°C, more preferentially between 10°C and 20°C.

The following stage in the manufacturing process consists in producing a second emulsion, where a first component will be the system of the first emulsion constituted by the aqueous phase, containing the active peptide, micro-emulsioned into the oily phase composed by the polymer dissolved in an organic solvent; and the second component is a vehicle aqueous phase, consisting in an aqueous solution of a hydrophilic protective colloid with tensoactive power, which is prepared for this intention. As the protective colloid, polyvinyl alcohol (PVA) is adopted. The result of this operation will be the formation of a complex emulsion water/oil/water from which the microcapsules containing the active peptide can be made. In the process of this invention, the agitation intensities are adjusted in each intensive agitation equipment, independently one of each other, and independently from the feed flows.

Several parameters have an influence on the preparation of this second emulsion. In this way, the chemical composition of the vehicle aqueous phase, the mass proportion of the same respect to the first emulsion, the temperature conditions and the agitation strength, are essential variables which may be controlled to obtain a microcapsules population with a size range that has an elevated mass composition of particles between 1 and 75 microns.

In a preferred form of the present invention, the vehicle aqueous phase is prepared by adding to the water, as hydrophilic protective colloid, polyvinyl alcohol (PVA) with an apparent viscosity of 25 to 50 centipoises, measured in 4% in weight aqueous solution and temperature of 20°C; with an hydrolysis grade between 85% and 89%, and a concentration between 0,1% and 1% in weight, preferentially between 0,2% and 0,4%. The presence of polyvinyl alcohol (PVA), as a hydrophilic protective colloid and tensoactive agent simultaneously, ensures the stability and a low particle aggregation, which allows positive results in microcapsules formation. The optimal temperature to produce this second emulsion is between 10°C and 30°C, more preferentially between 12°C and 20°C.

To produce this second complex water/oil/water emulsion type, a homogenizing device, a closed to ambient intensive agitation equipment, with cylinder shape, and using as agitation element an assembly rotor-stator with striaes. To this intensive agitation equipment the first emulsion and the vehicle aqueous phase arrive by two different dos-age pumps.

Referring to the agitation conditions, it is convenient to make this second emulsion operating the rotor at high speed. For a 17,5 mm of diameter rotor, rotating against a stator with striaes, it has been concluded, after a number of assays, that the rotor angular speed range may be between 10000 and 25000 rpm, or preferentially between 14000 and 18000 rpm, for a total entrance flow of the first emulsion plus the vehicle aqueous phase between 500 and 10000 ml/min. These angular speeds of the rotor may be expressed, in a more general way and considering the possibilities of procedure scaling in terms of rotor peripheral speed, which must be higher than 9 m/sec. to obtain the desired results. Moreover, the rotor rotating speed is controlled independently from the other variables of the process, which allows an adjusted regulation of the desired process condition. On the other side, the phases residence time, in this second intensive agitation equipment, to make the second water/oil/water emulsion type must be less than 1 second.

In order to complete the conditions assembly of this second emulsion in the method of the present invention, the mass ratio between the vehicle aqueous phase and the first emulsion must be between 30 and 80 times; more preferentially between 35 and 55. The temperature to make this water/oil/water emulsion must be controlled between 10°C and 30°C.

The procedure consists in adding the first micro emulsion containing the active peptide, in a continuous form, to a continuous flow of the aqueous phase with the tensoactive; phases are mixed rapidly in the agitation equipment applying a great stress with the assembly rotor-stator that induces the microcapsules formation, which in closed contact with the aqueous phase lose the superficial methylene chloride and are transformed, from a water/oil/water emulsion, into microcapsules with a rigidity able to maintain in suspension form without producing aggregates and with a particle size distribution that, once the temperature and the mass proportions of the both phases components are fixed, is determined by the rotor speed. From this rotor-stator assembly, which also acts as an centrifugal pump, the complex emulsion is sent to an evaporation recipient where, by diminishing gradually the pressure until values between 30 and 80 Torr, or more preferentially between 40 and 60 Torr, most of methylene chloride is eliminated with the consequent hardening of microcapsules that, in this conditions, reach so rigidity that they are able to support centrifugation without agglomerate.

Figure 1 illustrates some technical details concerning the dominion of this invention, which are described next. The closed recipient (7) contains the active peptide aqueous solution, while (8) is the container of the polymer in an organic solvent solution. By both dosage pumps, (3) for aqueous phase and (4) for oily phase, constant and controlled flow of the two phases are injected inside the intensive agitation equipment (1), where the first water/oil emulsion is produced. A detail that stands out as a part of this invention, is that the aqueous phase enters into the agitation equipment by a central tube, which ends at a distance not greater than 20 mm, or more preferentially not greater than 10 mm, while the oily phase enters by an external coaxial conduct. Both feed entrances reach the intensive agitation equipment in front of the agitation element.

The dosage pumps (3), (4), (5), and (6) are adjustable flow pumps independently, by own control devices, generally of low flow. In the context of the present invention, and depending on the scale of production, pumps (3) and (4) may be gears at adjustable speed type, or stainless steel piston type moved by a sin fin screw connected to a speed reductor of variable ratio with a synchronic or controlled speed motor.

From the intensive agitation equipment (1), that operates totally closed, the first water/oil emulsion pass through a heat exchanger (12) where it is cooled at a preestablished temperature, and it is discharged to the closed recipient (9) which works as feeder of that emulsion to form the second emulsion. The recipient (10) acts as a closed place of preparation and container of vehicle aqueous phase with the hydrophilic protective colloid dissolved. From these recipients the first emulsion, by pump (5), and the vehicle aqueous phase with pump (6), are fed in continuously way, and then enter the intensive agitation equipment (2) where the second water/oil/water emulsion is produced.

Like in case of the first intensive agitation equipment, the injection points of first emulsion and vehicle aqueous phase, so as the distance between that injection point and the rotor are high incidence factors in the successful manufacturing of the second emulsion process. In this invention dominion, it is established that is convenient to introduce the first water/oil emulsion by a central tube, and the vehicle aqueous phase by a coaxial annular tube, and that the central tube ends in front of equipment rotor (2), at a distance preferentially not greater than 20 mm.

The dosage pump (5), according to process scale, may be controlled speed gears type, or stainless steel piston type moved by a sin fin screw connected to a speed reducing of variable ratio with a synchronic or controlled speed motor. Pump (6) may be, among others, an adjustable peristaltic type, which in the context of the present invention, must have a flow greater than 400 ml/min.

The second water/oil/water emulsion type with the active peptide microcapsules already partially formed, is transferred by the action of the agitator pumping (2) to a closed recipient (11) provided with the following elements: a low speed marine type agitator, preferentially of not more than 150 rpm, or a magnetic agitator; a thermostat to keep the temperature between 10°C and 40°C; a connection with an automatic valve to an adjustable vacuum system (13), and a closed discharge conduct, with valve, to discharge the aqueous phase with the micro particles in suspension, to a sieving closed equipment. In this recipient (11), the final organic solvent evaporation and the final microcapsules consolidation, are produced. With the purpose to prevent the possible foam formation in the organic solvent evaporation process, this recipient must not be filled with liquid over 60% of its total volume.
The microcapsules in suspension are sieved through a 200 mesh that allows passing less than 75 microns of size particles. The material lost for a greater size than 75 microns is less than 10% and generally less than 5%, a very little value compared with the results of experiences of emulsions made into discontinuous reactors where the adding of the first emulsion to the aqueous phase is made without greater cares respect to the point of injection, and where the results for material loses of more than 75 microns of size, can rise more than 30%.

The suspension is now centrifuged into continuous equipment where the microcapsules of greater size are precipitated for their recollection, after being washed with distilled water, until the residue of protective colloid and peptide that has not been entrapped, is eliminated. The lost values in this case are very related to the peptide entrapped in the microcapsules, which generally is between 80% and 90%. Adjusting the centrifuge speed and the suspension flow it is possible to eliminate microcapsules of very little size and thinly divided polymer. Then, this separation process is preferred to the filtration one which does not eliminate most of the little size particles that can increase the burst effect in the final product.

In this way, the active peptide lost during this global procedure, respect to the quantity used as raw material, is less than 30%.

Once the washing is finished, the microcapsules are withdrawn from the centrifuge, resuspended in distilled water, and discharged into a provisional staying recipient.

By maintaining the microcapsules in this inert aqueous medium, and homogeneously by agitation, the actual quantity of active peptide is valued by HPLC. The necessary quantity of an aqueous solution of lyophilization excipient that facilitates the next lyophilizing operation and its final dryness, is added, being able to use lactose, polyvinylpyrrolidone, etc. For this intention, mannitol use has been found appropriated, in concentrations from about 0,1% to 5%, preferentially between 0,8% and 1,5% in weight referred to the total suspension.

The microcapsules aqueous suspension is fractionated by aseptic procedure in adequate containers for the active peptide. All of this process step is made in closed system and maintaining the agitation on the aqueous medium that contains the microcapsule, to ensure the suspension homogeneity.

This mentioned adequate recipients might be little dose vials, which after lyophilization are sealed to obtain the final product already packaged for consume, or greater size containers that, after lyophilization, allow to obtain the product in bulk as dried powder.

In this conditions, the operation of freezing the recipients contents is carried out into an orbital agitation freezer device, made to put the recipients containing the suspension, over a tray on which a circulating movement is produced. because the radius is less or equal to the recipient base radius that contains the microcapsules suspension. The support base of the tray is a refrigerating plate that by refrigerant fluid circulation may be cooled to temperatures between -20°C and -80°C, or more preferentially to temperatures between -30°C and -60°C. By this device and method, developed as a part of the present invention, the microcapsules suspension is frozen with the less possible particles deposit in the bottom of each vial. This procedure enormously facilitates the microcapsules lyophilization process and final dryness, producing a lyophilized material of greater homogeneity that improves considerably the reconstitution process of the microcapsules suspension, making this reconstitution to be instantaneous, avoid the microcapsules agglomeration during more time, and improves their pharmaceutical quality. The device is shown in figure 2 of the present document. In a preferred form of the present invention, the freezing process with orbital agitation is made gradually from a temperature of 15°C until a final one of -30°C, during a total estimated time between 5 min. and 60 min, or more preferentially between 15 min. and 30 min.
Figure 2 corresponds to a scheme of the orbital agitation freezer for the already fractionated micro-particles. The stainless steel plate (17) which is supported to a fixed structure, that does not appear in the scheme, by springs, has refrigerating fluid circulation in its inside, it is connected by the entering tubes (16) and exit tubes (22), connected by hoses to an programmed refrigeration external equipment which sends a refrigerating fluid to the plate. The flasks containing the microcapsules suspension with their final dosage are put over the plate, and they are covered with a sterilized polycarbonate box (21). The eccentric system (18) is in the plate (17) and it is connected to a adjustable speed motor by the axis (19). For the present invention intention, the eccentric disc (18) is made so that the orbital movement of the platform presents a rotating radius lesser than the bottom radius of the recipients put over the tray and the motor speed is adjusted, according to each case, between 20 rpm and 50 rpm. In the case that the recipients are not cylindrical, it is understood the radius of the circle in which the base is circumscribed.

The following step is the microcapsules lyophilizing, into the recipients where they have been fractionated, by an extensively known procedure in the technique, where the product might not be warmed up over the softening temperature of the polymer used. As the final stage of the continuous process proposed in the present invention, the vials are closed under nitrogen atmosphere into the lyophilizer.

In order to obtain the manufacturing of a determined quantity of microcapsules, this continuous procedure is made during a fixed time. Along that whole time, microcapsules maintaining practically the same size, the same particle diameter dispersion, and the same active peptide concentration are obtained, from the beginning to the end of the process, when the desired quantity of the product is obtained.

### EXAMPLE 1: Active peptide microencapsulation with rotor-stator type agitator.

Pharmaceutical raw materials that meet with the quality requirements included in the pharmacopoeia are used. The procedures are made under aseptic conditions, and under laminar flow class 100.
14.5 g of leuprolide acetate with a purity grade greater than 99%, with 2.29 g of gelatin, type B, 75 Blooms from bovine origin, are aseptically lyophilized. The lyophilized gelatin and the leuprolide are dissolved into 26 ml of water, warmed up to dissolve at 60°C and they are taken to a recipient for dosage (7). On the other side, 334 g of a PLGA in methylene chloride solution (this solution is prepared with 130 g of PLGA 75:25, MW: 14000 D. analyzed by gel permeation chromatography with polystyrene standards and 162 ml of methylene chloride) is put into the recipient for dosage (8). They are dosed by their respective dosage pumps, piston with screw sin fin type, being 250 ml/min the total flow entrance to the first intensive agitation equipment, being 7.8 the mass ratio of the oily polymer phase and the aqueous peptide phase. This continuous agitation equipment is an assembly rotor-stator (IKA), the peripheral rotor speed is 5 m/s. This first emulsion is refrigerated at 20°C when it passes through the refrigerator (12), going to the recipient (9), where it is accumulated until the necessary quantity desired for the process is obtained. The total entrance flow to the second intensive agitation equipment is 2330 ml/min. The mass ratio of the vehicle aqueous phase and the first water/oil emulsion is 67. The first emulsion is propelled by a dosage pump (5) piston with screw sin fin type in the water flow with polyvinyl alcohol of apparent viscosity of 35 centipoises measured in aqueous solution 4% in weight and temperature of 20°C, with an hydrolysis grade of 85%, and at 0.25% proportioned from the recipient (10) by a dosage pump (6), which for the example is peristaltic type, "Masterflex". This second agitation continuous equipment is an assembly rotor-stator (IKA), the peripheral rotor speed is 15 m/s. From there the water/oil emulsion is conducted to a recipient with magnetic agitation in which the methylene chloride is evaporated by diminishing the pressure during 90 minutes, reaching a pressure of 50 mm Hg during 45 minutes. The suspension so obtained, is taken to pass through a mesh 200 sift where 12.8 g (3.4%) of agglomerated microcapsules and them greater than 75 microns, are retained. The suspension is then conducted to a standard rotor of a continuous decant centrifuge (Beckman AVANTI J-25) by a dosage pump, at a 240 ml/m flow, with a rotation speed of 3000 rpm. It is washed with 100 ml of distilled water, the rotor is emptied, and the microcapsules are taken to a retention and dosage agitated recipient with a volume of 1750 ml, from where an approximately 1 ml aliquot is extracted to analyze by HPLC, as result a content of 5.60 mg of leuprolide acetate/ml is obtained, which implicates a dosage of 1.34 ml, to obtain doses of 7.5 mg after adding 123.2 ml of an sterile solution of mannitol 15% weight/volume. In each vial 1.34 ml are dosed, and 1380 vials are obtained from the theoretical amount of 1886 doses, according to the consumed leuprolide acetate quantity. So, the lost leuprolide acetate during the complete process, respect to the used quantity as raw material, is 26.8%. These vials are put in a lyophilizing tray, which is then put over the plate that is gradually refrigerated until a temperature of -50°C, while the eccentric orbital agitator moves at an orbital agitation speed of 120 rpm during a time of 30 minutes. Finished this operation, the tray is put into the lyophilizer and it is lyophilized until a pressure lesser than 10 microns, refrigerating at -40°C during 6 hours; at -5°C during 10 hours; at 0°C during 10 hours, and at 25°C during 90 hours, so to obtain a water residue grade lesser than 1%, and a methylene chloride contents less than 33 ppm. The vacuum is broken with sterile nitrogen and the vials are closed into the lyophilization equipment. The lyophilized product is sealed and it is stored at room temperature and protected from light, for its later analysis.

The microcapsules obtained in this example were put under to a particle diameter distribution by laser interferometry analysis, that gave the results shown in figure 3. A 15 microns diameter rate profile, and a diameter fraction greater than 75 microns, less than 2.5%, is observed.

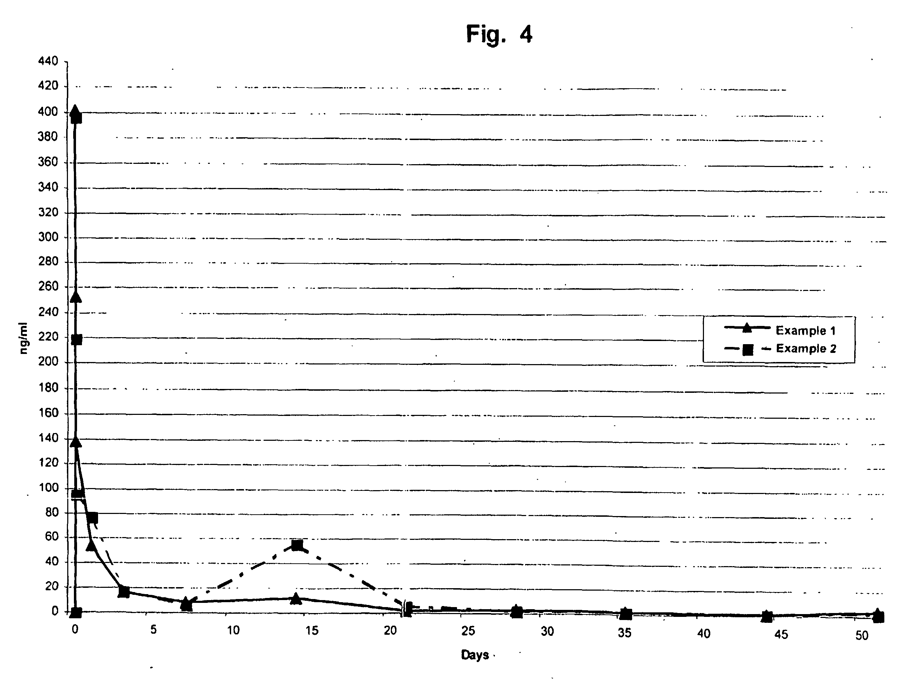

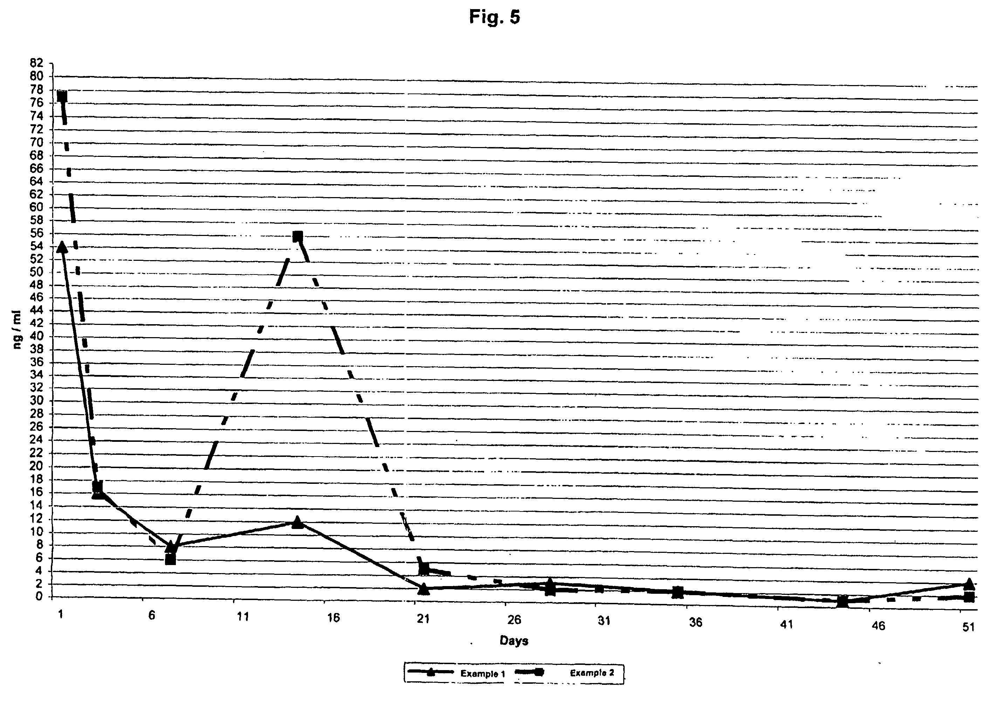

Also, in vivo test were made, where the leuprolide acetate kinetic release was evaluated, in Wistar rats by RIA analysis, with a leuprolide antibody developed by the technique according to "I Yamazaki and H. Okada, Endocrinol. Jpn., 27 (1980) 593-605". A release profile like in figure 4 was obtained, and in an amplified scale is seen in figure 5.

### EXAMPLE 2: Active peptide microencapsulation with sonotrode type agitator in water/oil emulsion; and rotor-stator in water/oil/water emulsion.

1.84 g of leuprolide acetate with 327 mg of gelatin, type B,75 Blooms from bovine origin, are aseptically lyophilized. The lyophilized gelatin with the leuprolide are dissolved in 3.3 ml of water, is warmed up to dissolve at 60°C, and they are taken to the recipient (7). Other side, 43.47 g of a PLGA in methylene chloride solution (this solution is prepared with 17.36 g of PLGA 75:25, MW: 14000 D. and 21.7 ml of methylene chloride) is put in the recipient (8). Both solutions must pass simultaneously, by two dosage pumps piston with screw sin fin type at a 35 ml/min through the continuous equipment (Dr Hielscher Gmbh) thermostatised at 18°C, while it is agitated with a 2 mm sonotrode at a power of 40 W, at 30 KHz (Ultrasonic processor UP 50 H, Dr. Hielscher Gmbh). The resulting water/oil emulsion is refrigerated until 20°C and arrives to a recipient (9). Then, is injected with a dosage pump, piston con screw sin fin type, along with a water flow with polyvinyl alcohol 0.25% that is incorporated from the recipient (10) by a dosage pump, peristaltic type, Masterflex. These flows enter coaxially to the second intensive agitation equipment that has an assembly rotor-stator (IKA). The peripheral rotor speed is 15 m/sec. The entering flow to this equipment is 2330 ml/m. The water/oil/water emulsion so obtained is continuously conducted to a recipient (11) with magnetic agitation in which the methylene chloride is evaporated during 90 minutes, reaching a pressure of 50 mmHg during 45 minutes. This suspension passes through a mesh 200 sieve, where 1.9 g (3.9%) of agglomerated microcapsules and those greater than 75 microns, are retained. The suspension is then conducted by a dosage pump, at a 240 ml/min. flow, to a continuous centrifuge (Beckman AVANTI J-25) that works at a rotation speed of 3000 rpm. There it is washed with a volume of 1000 ml of distilled water, the rotor is emptied, and the microcapsules are carried to a dosage machine with a volume of 255 ml, from where, an aliquot of approximately 1 ml is taken, to analyze the content of leuprolide acetate by HPLC, as a result a content of 5.62 mg of leuprolide acetate per ml is obtained. Resulting in a dosage of 1.42 ml to obtain 7.5 mg doses after adding 16.8 ml of a Mannitol solution 15% w/v. In final vials, 1.42 g are dosed and 185 dosed samples are obtained, from the theoretical 245, according the Leuprolide quantity used. So, the lost of leuprolide acetate during the complete process, respect to the used quantity as raw material, is 23.2.%. These vials containing the microcapsules suspension are put into the orbital agitation freezer at 20 rpm, that refrigerates gradually until -50°C during a time of 25 minutes; then, it is lyophilized following the same sequence that in example 1, to obtain a humidity grade lesser than 1% and a methylene chloride content lesser than 33 ppm. The vacuum is broken with sterile nitrogen, and the closure is made inside the lyophilizer. The lyophilized product is sealed, and stored at room temperature and protected from light, for its later analysis.

### EXAMPLE 3: Active peptide microencapsulation with rotor-stator type agitator.

17.83 g of goserelin acetate with 2.89 g of gelatin, type B, 75 Blooms from bovine origin, are aseptically lyophilized. The lyophilized gelatin with the goserelin are dissolved in 32 ml of water, is warmed up to dissolve at 60°C, and they are taken to the dosage recipient (7). On the other side, 332 g of a PLA of MW 20000 in methylene chloride solution (this solution is prepared with 154 g of PLA and 192 ml of methylene chloride) is put in the dosage recipient (8). They are dosed by their respective dosage pumps with a total flow of 100 ml/min, and a ratio of oily and aqueous phases of 6, to the first intensive agitation equipment, like in example N¼ 1. The resulting emulsion is cold at 20°C by passing through the refrigerator (12), following to the recipient (9), then injected into the water flow with 0.25% polyvinyl alcohol. The total entrance flow to the second intensive agitation equipment is 2730 ml/min, given by a peristaltic dosage pump, Watson-Marlow. The second emulsion was made in a continuous equipment (IKA) with the homogenizer (IKA) at a peripheral speed of 15 m/s, and conducted to a magnetic agitation reactor in which the methylene chloride is evaporated by diminishing the pressure during 1 hour and a half, reaching a pressure of 50 mmHg during 45 minutes. The suspension so obtained is passed through a 200 mesh sieve, where 12.8 g (3.3%) of agglomerated microspheres, and those greater than 75 microns, are retained. The suspension is then conducted to a standard rotor from a continuous centrifuge (Beckman AVANTI J-25) by a dosage pump at a flow of 240 ml/m, with a rotation speed of 3000 rpm. It is washed with 1000 ml of distilled water, the rotor is emptied taking the microcapsules to a volume of 3500 ml, form where an aliquot of 1 ml is taken to be analyzed by HPLC, from where a content of 3.75 mg of goserelin acetate/ml results, which implicates a dosage of 1.00 ml to obtain doses of 3.6 mg after adding 160.4 ml of a 15% w/v mannitol solution. 1 ml into each vial is dosed, and 3652 dosed samples are obtained from the theoretical 4952, according to the used quantity of goserelin (73.7%), these are put over the plate refrigerated at -50°C, with its tray for lyophilization over the circular agitator that moves at a speed of 120 cycles/minute, during a time of 30 minutes. Then the tray is put into the lyophilizer and it is lyophilized following the same sequence of the example 1, to obtain a humidity grade lesser than 1%, and a content of methylene chloride lesser than 33 ppm. The vacuum is broken with sterile nitrogen, and the vials are closed into the lyophilizer. The lyophilized product is sealed, and stored at room temperature and out of the reach of light, for its later analysis.

## Claims

1. A process for producing sustained release microcapsules of water soluble peptides, usable in injectable preparations and with adjustable release periods between 1 and 18 weeks, in which an aqueous solution of an active peptide containing a retention substance that is continuously emulsioned into an oily solution of a biodegradable polymer dissolved in an very slight soluble in water organic solvent, using a first intensive agitation equipment, closed to ambient, fed by dosage machines; this first water/oil emulsion, after being refrigerated, is fed by a dosage machine, to a second intensive agitation equipment closed to ambient, where it is emulsioned, in continuous way, into a vehicle aqueous phase containing a protective hydrophilic colloid, which is fed by a continuous dosage machine; this second complex water/oil/water emulsion type is transported in continuous way to a closed recipient where the solvent is evaporated by pressure reduction, producing the microcapsules consolidation; **characterized by** said microcapsules, obtained by a double and continuous W/O/W emulsification, are processed in wet way: they are sieved in suspension; they are centrifuged; they are washed with water; and they are fractionated into adequate recipients, dispersed into an aqueous medium containing a lyophilization excipient; then they are frozen in an orbital agitation freezer until temperatures less than -20 °C and they are lyophilized into the same recipients.

2. A process for producing sustained release microcapsules, as claimed in claim 1, **characterized by** the microcapsules have a size distribution ranging from 0.5 to 100 microns, being the discard percentage due to particle size greater than 75 microns, less than 10%.

3. A process for producing sustained release microcapsules as claimed in claim 1, **characterized by** the dosage machines are adjustable flow pumps.

4. A process for producing sustained release microcapsules as claimed in claim 1, **characterized by** the first intensive agitation equipment, used for the first emulsion formation, works with residence time lesser than 7 seconds.

5. A process for producing sustained release microcapsules, as claimed in claim 1, **characterized by** the first intensive agitation equipment is cylindrical, and uses as agitator element an assembly rotor-stator with strives.

6. A process for producing sustained release microcapsules, as claimed in claim 5, **characterized by** the rotor peripheral speed of the first intensive agitation equipment is greater than 3 m/sec.

7. A process for producing sustained release microcapsules, as claimed in claim 1, **characterized by** the first intensive agitation equipment presents as the agitator element a sonotrode.

8. ,- A process for producing sustained release microcapsules, as claimed in claim 1, **characterized by** the second intensive agitation equipment used for the formation of the second water/oil/water emulsion presents as the agitator element an assembly rotor-stator with striaes.

9. A process for producing sustained release microcapsules, as claimed in claim 8, **characterized by** the assembly rotor-stator of the second intensive agitation equipment works with times of residence lesser than 1 second, and peripheral rotor speeds greater than 9 m/sec.

10. A process for producing sustained release microcapsules, as claimed in claim 1, **characterized by** the active peptide aqueous solution is emulsioned into the oily polymer phase, injecting both solutions coaxially to the agitator element axis, in the front face of the first intensive agitation equipment, with the aqueous solution entering by the inner tube at a distance from the agitator device not greater than 20 mm.

11. A process for producing sustained release microcapsules, as claimed in claim 1, **characterized by** the total feed flow to the first intensive agitation equipment is from about 30 to 500 ml/min.

12. A process for producing sustained release microcapsules, as claimed in claim 1, **characterized by** the mass ratio between the oily solution of the biodegradable polymer and the aqueous phase containing the active peptide at the entrance of the first intensive agitation equipment is from about 6 to 10.

13. A process for producing sustained release microcapsules, as claimed in claim 1, **characterized by** the total feed flow to the second intensive agitation equipment is from about 500 to 10000 ml/min.

14. A process for producing sustained release microcapsules, as claimed in claim 1, **characterized by** the first water/oil emulsion is emulsioned into the vehicle aqueous phase injecting both flows coaxially to the axis of the agitating element, in the front face of the second intensive agitation equipment, and with the water/oil emulsion entering by the inner tube, at a distance from the agitating device not greater than 20 mm.

15. A process for producing sustained release microcapsules, as claimed in claim 1, **characterized by** the mass ratio between the vehicle aqueous phase and the first water/oil emulsion, at the entrance of the second intensive agitation equipment, is from about 30 to 80.

16. A process for producing sustained release microcapsules, as claimed in claim 1, **characterized by** the mentioned retention substance is a gelatin with jellyfiant power, from about Bloom 75 to Bloom 100, type B, from bovine origin, which is added in the aqueous solution of the active peptide at a concentration ranging from 0% to 10% in weight.

17. A process for producing sustained release microcapsules, as claimed in claim 1, **characterized by** the biodegradable polymer is a copolymer of d, l-lactic acid and glycolic acid, of a molecular weight from about 10000 to 30000 Daltons.

18. A process for producing sustained release microcapsules, as claimed in claim 18, **characterized by** the monomers molar ratio, lactic:glycolic acid, range from 50:50 to 100:0.

19. A process for producing sustained release microcapsules, as claimed in claim 1, **characterized by** the organic solvent of biodegradable polymer is methylene chloride.

20. A process for producing sustained release microcapsules, as claimed in claim 1, **characterized by** the protective hydrophilic colloid of the vehicle aqueous phase is polyvinyl alcohol (PVA), of an apparent viscosity of 25 to 50 centipoises, measured in a 4% in weight aqueous solution, and temperature of 20 °C, with an hydrolysis grade from about 85% to 89% and a concentration from about 0.1% to 1% in weight.

21. A process for producing sustained release microcapsules, as claimed in claim 1, **characterized by** the lyophilization excipient is mannitol at a concentration from about 0.1 to 5% in weight referred to the total suspension.

22. A process for producing sustained release microcapsules, as claimed in claim 1, **characterized by** the dispersed microcapsules into an aqueous phase, and fractionated in the recipients for the final dosage, are frozen into an orbital agitation freezer until temperatures below - 20°C, during a time from about 5 to 60 min.

23. A process for producing sustained release microcapsules, as claimed in claim 1, **characterized by** the orbital agitation freezer used for freezing, is a refrigerated plate with an orbital circular movement where the rotation radius is lesser or equal to the radius of the adequate recipients base, which contains the aqueous dispersion of microcapsules.

24. A process for producing sustained release microcapsules, as claimed in claim 1, **characterized by** the agitator device makes an orbital circular movement, with a rotation speed from about 20 to 50 r.p.m.

25. A process for producing sustained release microcapsules, as claimed in claim 1, **characterized by** the centrifugation, washing, microcapsules dispersion into an aqueous medium, and freezing with agitation operations are made in a closed system.

26. A process for producing sustained release microcapsules, as claimed in claim 1, **characterized by**, while it is being made, microcapsules maintain practically the same size, the same particle diameter dispersion, and the same active peptide concentration are obtained, during the whole time that the procedure is prolonged.

27. A process for producing sustained release microcapsules, as claimed in claim 1, **characterized by** the solution feed flows or dispersions that enter to the intensive agitation equipments, are independently adjusted.

28. A process for producing sustained release microcapsules, as claimed in claim 1, **characterized by** the agitation intensities are adjusted in each intensive agitation equipment, independently one of each other, and independently from the feed flows.

29. A process for producing sustained release microcapsules, as claimed in claim 1, **characterized by** the active peptide is leuprolide acetate.

30. A process for producing sustained release microcapsules, as claimed in claim 1, **characterized by** the active peptide is goserelin acetate.

31. A process for producing sustained release microcapsules, as claimed in claim 1, **characterized by** the active peptide is nafarelin acetate.

32. A process for producing sustained release microcapsules, as claimed in claim 1, **characterized by** the active peptide is triptorelin acetate.

33. A process for producing sustained release microcapsules, as claimed in claim 1, **characterized by** the active peptide is buserelin acetate.

34. A process for producing sustained release microcapsules, as claimed in claim 1, **characterized by** the active peptide lost percentage during the all process, respecting to the used quantity as raw material, is less than 30%.

35. A process for producing sustained release microcapsules, as claimed in claim 1, **characterized by** the adequate recipients are little dose vials, which after lyophilization are sealed to obtain the final product for consume.

36. A process for producing sustained release microcapsules, as claimed in claim 1, **characterized by** the adequate recipients are containers that allow, after lyophilization, to obtain the product in bulk.

37. A process for producing sustained release microcapsules, as claimed in claim 1, **characterised by** the microcapsules powder resulting from this procedure does not present agglomeration for a time, and the suspension reconstitution to be injected is instantaneous.

## Patentansprüche

1. Verfahren zur Herstellung von Mikrokapseln mit verzögerter Freisetzung von wasserlöslichen Peptiden, verwendbar in injizierbaren Zusammensetzungen und mit einstellbaren Abgabezeiträumen zwischen einer und achtzehn Wochen, in welchem eine wässrige Lösung eines aktiven Peptids enthaltend eine Retentionssubstanz, die kontinuierlich in eine ölige Lösung eines biologisch abbaubaren Polymers emulgiert wird, gelöst in einem sehr leicht in Wasser löslichen organischen Lösungsmittel, unter Verwendung einer ersten nach außen abgeschlossenen Anlage zum intensiven Rühren, Zuführung mittels Dosiermaschinen; diese erste Wasser-/Öl-Emulsion wird, nachdem sie eingefroren ist, mittels einer Dosiermaschine zu einer zweiten nach außen abgeschlossenen Anlagen zum intensiven Rühren transportiert, wo sie kontinuierlich in eine Vehikel Wasserphase, enthaltend ein schützendes hydrophiles Kolloid emulgiert wird, welches durch eine kontinuierliche Dosiermaschine transportiert wird; dieser zweite komplexe Wasser-/Öl-/Wasser-Emulsionstyp wird kontinuierlich zu einem geschlossenen Rezipienten transportiert, wo das Lösungsmittel durch Druckreduktion entfernt wird, wobei die Mikrokapselverfestigung erzeugt wird; **dadurch gekennzeichnet, dass**
die durch eine doppelte und kontinuierliche W-/O-/W-Emulgierung erhaltenen Mikrokapseln auf nasse Art bearbeitet werden:
sie werden gesiebt in Suspension;
sie werden zentrifugiert;
sie werden mit Wasser gewaschen; und
sie werden in geeignete Rezipienten fraktioniert, dispergiert in ein wässriges Medium enthaltend einen Gefriertrocknungs-Arzneimittelträger; dann
werden sie in einem kreisförmigen Rühr-Gefrierapparat bei Temperaturen von weniger als -20°C getrocknet und
sie werden in die gleichen Rezipienten gefriergetrocknet.

2. Verfahren zur Herstellung von Mikrokapseln mit verzögerter Freisetzung wie gemäß Anspruch 1 beansprucht, **dadurch gekennzeichnet, dass** die Mikrokapseln eine Größenverteilung im Bereich von 0.5 bis 100 Mikrometer haben und der Ausschussprozentsatz aufgrund einer Partikelgröße von weniger als 75 Mikrometer, weniger als 10 % ist.

3. Verfahren zur Herstellung von Mikrokapseln mit verzögerter Freisetzung, wie in Anspruch 1 beansprucht, **dadurch gekennzeichnet, dass** die Dosierungsmaschinen, einstellbare Vorlaufpumpen sind.

4. Verfahren zur Herstellung von Mikrokapseln mit verzögerter Freisetzung, wie in Anspruch 1 beansprucht, **dadurch gekennzeichnet, dass** die erste, für die erste Emulsionsbildung verwendete Anlage zum intensiven Rühren mit einer Aufenthaltszeit von weniger als 7 Sekunden arbeitet.

5. Verfahren zur Herstellung von Mikrokapseln mit verzögerter Freisetzung, wie in Anspruch 1 beansprucht, **dadurch gekennzeichnet, dass** die erste Anlage zum intensiven Rühren zylindrisch ist und als Rührelement eine Baugruppe Rotor-Stator mit Streben (strives) benützt.

6. Verfahren zur Herstellung von Mikrokapseln mit verzögerter Freisetzung, wie in Anspruch 5 beansprucht, **dadurch gekennzeichnet, dass** die Rand-Rotorgeschwindigkeit der ersten Anlage zum intensiven Rühren größer als 3 m/Sek. ist.

7. Verfahren zur Herstellung von Mikrokapseln mit verzögerter Freisetzung, wie in Anspruch 1 beansprucht, **dadurch gekennzeichnet, dass** die erste Anlage zum intensiven Rühren als Rührelement eine Sonotrode aufweist.

8. Verfahren zur Herstellung von Mikrokapseln mit verzögerter Freisetzung, wie in Anspruch 1 beansprucht, **dadurch gekennzeichnet, dass** die zweite zur Bildung der zweiten Wasser-/Öl-/Wasser-Emulsion verwendete Anlage zum intensiven Rühren als das Rührelement eine Anordnung Rotor-Stator mit Streben aufweist.

9. Verfahren zur Herstellung von Mikrokapseln mit verzögerter Freisetzung, wie in Anspruch 8 beansprucht, **dadurch gekennzeichnet, dass** die Anordnung Rotor-Stator der zweiten Anlage zum intensiven Rühren mit Verweilzeiten von weniger als 1 Sek. und Rand-Rotorgeschwindigkeiten größer als 9 m/Sek. arbeitet.

10. Verfahren zur Herstellung von Mikrokapseln mit verzögerter Freisetzung, wie in Anspruch 1 beansprucht, **dadurch gekennzeichnet, dass** die wässrige Lösung aktiver Peptide in die Ölpolymerphase emulgiert wird, wobei beide Lösung koaxial zu den Rührelementachsen auf der Vorderseite der ersten Anlage zum intensiven Rühren eingespritzt werden, wobei die Entfernung der innere Röhre für die wässrige Lösung zur Rührvorrichtung nicht größer als 20 mm ist.

11. Verfahren zur Herstellung von Mikrokapseln mit verzögerter Freisetzung, wie in Anspruch 1 beansprucht, **dadurch gekennzeichnet, dass** die gesamte Flussrate zur ersten Anlage zum intensiven Rühren zwischen etwa 30 bis 500 ml/Min. ist.

12. Verfahren zur Herstellung von Mikrokapseln mit verzögerter Freisetzung, wie in Anspruch 1 beansprucht, **dadurch gekennzeichnet, dass** das Massenverhältnis zwischen der öligen Lösung des bioabbaubaren Polymers und der wässrigen Phase, die das aktive Peptid enthält, beim Eingang der ersten Anlage zum intensiven Rühren von etwa 6 bis 10 ist.

13. Verfahren zur Herstellung von Mikrokapseln mit verzögerter Freisetzung, wie in Anspruch 1 beansprucht, **dadurch gekennzeichnet, dass** die gesamte Flussrate zur zweiten Anlage zum intensiven Rühren von etwa 500 bis 10 000 ml/Min. ist.

14. Verfahren zur Herstellung von Mikrokapseln mit verzögerter Freisetzung, wie in Anspruch 1 beansprucht, **dadurch gekennzeichnet, dass** die erste Wasser-/Öl-Emulsion in die wässrige Trägerphase emulgiert wird, wobei beide Ströme koaxial zu der Achse des Rührelements, auf der Vorderseite der zweiten Anlage zum intensiven Rühren und unter Eintritt der Wasser-/Öl-Emulsion durch die innere Röhre bei einer Entfernung von nicht mehr als 20 mm von der rührenden Vorrichtung, eingespritzt werden.

15. Verfahren zur Herstellung von Mikrokapseln mit verzögerter Freisetzung, wie in Anspruch 1 beansprucht, **dadurch gekennzeichnet, dass** das Massenverhältnis zwischen der wässrigen Trägerphase und der ersten Wasser-/Öl-Emulsion beim Eintritt von der zweiten Anlage zum intensiven Rühren von etwa 30 bis 80 ist.

16. Verfahren zur Herstellung von Mikrokapseln mit verzögerter Freisetzung, wie in Anspruch 1 beansprucht, **dadurch gekennzeichnet, dass** die erwähnte Retensionssubstanz eine Gelatine mit Gelierungskraft von etwa Bloom 75 bis Bloom 100, Typ B, Herkunft von Rindern ist, welche in die wässrigen Lösung des aktiven Peptids bei einer Konzentration im Bereich von 0 bis 10 Gew.-% gegeben wird.

17. Verfahren zur Herstellung von Mikrokapseln mit verzögerter Freisetzung, wie in Anspruch 1 beansprucht, **dadurch gekennzeichnet, dass** das bioabbaubare Polymer ein Copolymer von d,l-Milchsäure und Glykolsäure mit einem Molekulargewicht von etwa 10 000 bis 30 000 Dalton ist.

18. Verfahren zur Herstellung von Mikrokapseln mit verzögerter Freisetzung, wie in Anspruch 17 beansprucht, **dadurch gekennzeichnet, dass** das Monomer-Molverhältnis Milchsäure : Glykolsäure im Bereich von 50 : 50 bis 100 : 0 ist.

19. Verfahren zur Herstellung von Mikrokapseln mit verzögerter Freisetzung, wie in Anspruch 1 beansprucht, **dadurch gekennzeichnet, dass** das organische Lösungsmittel des bioabbaubaren Polymers Methylenchlorid ist.

20. Verfahren zur Herstellung von Mikrokapseln mit verzögerter Freisetzung, wie in Anspruch 1 beansprucht, **dadurch gekennzeichnet, dass** das schützende hydrophile Kolloid der wässrigen Trägerphase Polyvinylalkohol (PVA) mit einer scheinbaren Viskosität von 25 bis 50 Centipoise, gemessen in einer 4 Gew.-%igen wässrigen Lösung und Temperaturen von 20°C mit einem Hydrolysegrad von etwa 85 bis 89 % und einer Konzentration von etwa 0.1 bis 1 Gew.-% ist.

21. Verfahren zur Herstellung von Mikrokapseln mit verzögerter Freisetzung, wie in Anspruch 1 beansprucht, **dadurch gekennzeichnet, dass** der Gefriertrocknungsarzneimittelträger Mannitol bei einer Konzentration von etwa 0.1 bis 5 Gew.-% bezogen auf die gesamte Suspension ist.

22. Verfahren zur Herstellung von Mikrokapseln mit verzögerter Freisetzung, wie in Anspruch 1 beansprucht, **dadurch gekennzeichnet, dass** die in eine wässrige Phase dispergierten und in die Rezipienten zur finalen Dosierung fraktionierten Mikrokapseln in einem kreisförmigen Rühr-Gefrierapparat bei Temperaturen unter -20°C während einer Zeit von etwa 5 bis 60 Minuten gefroren werden.

23. Verfahren zur Herstellung von Mikrokapseln mit verzögerter Freisetzung, wie in Anspruch 1 beansprucht, **dadurch gekennzeichnet, dass** der zum Einfrieren verwendete kreisförmige Rühr-Gefrierapparatar eine Gefrierplatte mit einer kreisförmigen zirkularen Bewegung ist, worin der Rotationsradius geringer oder gleich dem Radius der geeigneten Reziepienten-Grundfläche ist, welche die wässrige Dispersion der Mikrokapseln enthält.

24. Verfahren zur Herstellung von Mikrokapseln mit verzögerter Freisetzung, wie in Anspruch 1 beansprucht, **dadurch gekennzeichnet, dass** die Rührvorrichtung eine kreisförmige zirkulare Bewegung mit einer Rotationsgeschwindigkeit von etwa 20 bis 50 Umdrehungen pro Minute macht.

25. Verfahren zur Herstellung von Mikrokapseln mit verzögerter Freisetzung, wie in Anspruch 1 beansprucht, **dadurch gekennzeichnet, dass** die Zentrifugation, das Waschen, die Mikrokapsel Verteilung in ein wässriges Medium und das Gefrieren mit Rührarbeiten in einem geschlossenen System durchgeführt werden.

26. Verfahren zur Herstellung von Mikrokapseln mit verzögerter Freisetzung, wie in Anspruch 1 beansprucht, **dadurch gekennzeichnet, dass** während seiner Durchführung Mikrokapseln praktisch die gleiche Größe und die gleiche Teilchendurchmesser-Verteilung beibehalten und die gleiche Konzentrationen an aktivem Peptid während der ganzen Zeit, dass das Verfahren anhält, erhalten werden.

27. Verfahren zur Herstellung von Mikrokapseln mit verzögerter Freisetzung, wie in Anspruch 1 beansprucht, **dadurch gekennzeichnet, dass** die Lösungszuführungsströme oder Dispersionen, welche die Anlagen zum intensiven Rühren erreichen, unabhängig angepasst werden.

28. Verfahren zur Herstellung von Mikrokapseln mit verzögerter Freisetzung, wie in Anspruch 1 beansprucht, **dadurch gekennzeichnet, dass** die Rührintensitäten in jeder einzelnen Anlage zum intensiven Rühren unabhängig voneinander und unabhängig von den Flussraten eingestellt werden.

29. Verfahren zur Herstellung von Mikrokapseln mit verzögerter Freisetzung, wie in Anspruch 1 beansprucht, **dadurch gekennzeichnet, dass** das aktive Peptid Leuprolidacetat ist.

30. Verfahren zur Herstellung von Mikrokapseln mit verzögerter Freisetzung, wie in Anspruch 1 beansprucht, **dadurch gekennzeichnet, dass** das aktive Peptid Goserelinacetat ist.

31. Verfahren zur Herstellung von Mikrokapseln mit verzögerter Freisetzung, wie in Anspruch 1 beansprucht, **dadurch gekennzeichnet, dass** das aktive Peptid Nafarelinacetat ist.

32. Verfahren zur Herstellung von Mikrokapseln mit verzögerter Freisetzung, wie in Anspruch 1 beansprucht, **dadurch gekennzeichnet, dass** das aktive Peptid Triptorelinacetat ist.

33. Verfahren zur Herstellung von Mikrokapseln mit verzögerter Freisetzung, wie in Anspruch 1 beansprucht, **dadurch gekennzeichnet, dass** das aktive Peptid Buserelinacetat ist.

34. Verfahren zur Herstellung von Mikrokapseln mit verzögerter Freisetzung, wie in Anspruch 1 beansprucht, **dadurch gekennzeichnet, dass** der prozentuale Verlust des aktiven Peptids während des ganzen Verfahrens, bezogen auf die als Rohmaterial verwendete Menge weniger als 30 % ist.

35. Verfahren zur Herstellung von Mikrokapseln mit verzögerter Freisetzung, wie in Anspruch 1 beansprucht, **dadurch gekennzeichnet, dass** die geeigneten Rezipienten kleine Dosierungsfläschchen sind, welche nach Gefriertrocknung versiegelt werden, um das finale Produkt zum Konsum zu erhalten.

36. Verfahren zur Herstellung von Mikrokapseln mit verzögerter Freisetzung, wie in Anspruch 1 beansprucht, **dadurch gekennzeichnet, dass** die geeigneten Rezipienten Gebinde sind, die erlauben, nach Gefriertrocknung das Produkt im Ganzen zu erhalten.

37. Verfahren zur Herstellung von Mikrokapseln mit verzögerter Freisetzung, wie in Anspruch 1 beansprucht, **dadurch gekennzeichnet, dass** das aus diesem Verfahren resultierende Mikrokapselpulver eine Zeit lang keine Agglomeration aufweist, und dass die einzuspritzende Suspensionswiederherstellung augenblicklich ist.

## Revendications

1. Procédé de production de microcapsules à libération prolongée de peptides solubles dans l'eau, utilisables dans des préparations injectables et avec des périodes de libération ajustables comprises entre 1 et 18 semaines, dans lequel une solution aqueuse d'un peptide actif contenant une substance de rétention est émulsionnée en continu dans une solution huileuse d'un polymère biodégradable dissous dans un solvant organique très légèrement soluble dans l'eau, en utilisant un premier équipement d'agitation intensive, fermé à l'ambiante, alimenté par des machines de dosage ; cette première émulsion eau/huile, après avoir été réfrigérée, alimente par une machine de dosage un deuxième équipement d'agitation fermé à l'ambiante, où elle est émulsionnée, de manière continue, dans un véhicule en phase aqueuse contenant un colloïde protecteur hydrophile, qui est alimenté par une machine de dosage en continu ; ce deuxième complexe de type émulsion eau/huile/eau est transporté de manière continue jusqu'à un récipient fermé dans lequel le solvant est évaporé par réduction de pression, produisant la consolidation des microcapsules ; **caractérisé en ce que** lesdites microcapsules, obtenues par une émulsification double et continue eau/huile/eau, sont traitées à l'état humide : elles sont tamisées en suspension ; elles sont centrifugées ; elles sont lavées avec de l'eau et elles sont fractionnées dans des récipients adéquats, dispersées dans un milieu aqueux contenant un excipient de lyophilisation ; puis elles sont congelées dans un congélateur à agitation orbitale jusqu'à des températures inférieures à -20°C et elles sont lyophilisées dans les mêmes récipients.

2. Procédé de production de microcapsules à libération prolongée selon la revendication 1, **caractérisé en ce que** les microcapsules présentent une distribution de dimensions s'étendant de 0,5 à 100 microns, le pourcentage de rebut dû à une taille des particules supérieure à 75 microns, étant inférieur à 10 %.

3. Procédé de production de microcapsules à libération prolongée selon la revendication 1, **caractérisé en ce que** les machines de dosage sont des pompes à débit ajustable.

4. Procédé de production de microcapsules à libération prolongée selon la revendication 1, **caractérisé en ce que** le premier équipement d'agitation intensive, utilisé pour la formation de la première émulsion, fonctionne avec un temps de séjour inférieur à 7 secondes.

5. Procédé de production de microcapsules à libération prolongée selon la revendication 1, **caractérisé en ce que** le premier équipement d'agitation intensive est cylindrique et utilise, comme élément agitateur, un ensemble rotor - stator avec des stries.

6. Procédé de production de microcapsules à libération prolongée selon la revendication 5, **caractérisé en ce que** la vitesse périphérique du rotor du premier équipement d'agitation intensive est supérieure à 3 m/sec.

7. Procédé de production de microcapsules à libération prolongée selon la revendication 1, **caractérisé en ce que** le premier équipement d'agitation intensive présente comme élément de l'agitateur, une sonotrode.

8. Procédé de production de microcapsules à libération prolongée selon la revendication 1, **caractérisé en ce que** le deuxième équipement d'agitation intensive utilisé pour la formation de la deuxième émulsion eau/huile/eau présente comme élément agitateur un ensemble rotor - stator avec des stries.

9. Procédé de production de microcapsules à libération prolongée selon la revendication 8, **caractérisé en ce que** l'ensemble rotor - stator du deuxième équipement d'agitation intensive fonctionne avec un temps de séjour inférieur à 1 seconde et des vitesses périphériques du rotor supérieures à 9 m/ sec.

10. Procédé de production de microcapsules à libération prolongée selon la revendication 1, **caractérisé en ce que** la solution aqueuse du peptide actif est émulsionnée dans la phase polymère huileuse, en injectant les deux solutions co-axialement par rapport à l'axe de l'élément d'agitation, dans la face frontale du premier équipement d'agitation intensive, la solution aqueuse entrant par le tube intérieur à une distance du dispositif agitateur qui n'est pas supérieure à 20 mm.

11. Procédé de production de microcapsules à libération prolongée selon la revendication 1, **caractérisé en ce que** le flux d'alimentation total jusqu'au premier équipement d'agitation intensive est d'environ 30 à 500 ml/min.

12. Procédé de production de microcapsules à libération prolongée selon la revendication 1, **caractérisé en ce que** le rapport en masse entre la solution huileuse du polymère biodégradable et la phase aqueuse contenant le peptide actif à l'entrée du premier équipement d'agitation intensive est d'environ 6 à 10.

13. Procédé de production de microcapsules à libération prolongée selon la revendication 1, **caractérisé en ce que** le flux d'alimentation total jusqu'au deuxième équipement d'agitation intensive est d'environ 500 à 10 000 ml/min.

14. Procédé de production de microcapsules à libération prolongée selon la revendication 1, **caractérisé en ce que** la première émulsion eau/huile est émulsionnée dans le véhicule en phase aqueuse en injectant les deux flux coaxialement par rapport à l'axe de l'élément d'agitation, dans la face frontale du deuxième équipement d'agitation intensive, et l'émulsion eau/huile entrant par le tube intérieur, à une distance du dispositif d'agitation qui n'est pas supérieure à 20 mm.

15. Procédé de production de microcapsules à libération prolongée selon la revendication 1, **caractérisé en ce que** le rapport de masse entre le véhicule en phase aqueuse et la première émulsion eau/huile, à l'entrée du deuxième équipement d'agitation intensive, est d'environ 30 à 80.

16. Procédé de production de microcapsules à libération prolongée selon la revendication 1, **caractérisé en ce que** la substance de rétention mentionnée est une gélatine avec une poudre gélifiante, de 75 Bloom à 100 Bloom environ, de type B, d'origine bovine, qui est ajoutée dans la solution aqueuse du peptide actif à une concentration de l'ordre de 0 % à 10 % en poids.

17. Procédé de production de microcapsules à libération prolongée selon la revendication 1, **caractérisé en ce que** le polymère biodégradable est un copolymère d'acide d,1-lactique et d'acide glycolique, d'un poids moléculaire d'environ 10000 à 30000 Daltons.

18. Procédé de production de microcapsules à libération prolongée selon la revendication 18, **caractérisé en ce que** le rapport molaire des monomères, acide lactique : acide glycolique, va de 50:50 à 100:0.

19. Procédé de production de microcapsules à libération prolongée selon la revendication 1, **caractérisé en ce que** le solvant organique du polymère biodégradable est le chlorure de méthylène.

20. Procédé de production de microcapsules à libération prolongée selon la revendication 1, **caractérisé en ce que** le colloïde protecteur hydrophile du véhicule en phase aqueuse est l'alcool polyvinylique (PVA), d'une viscosité apparente de 25 à 50 centipoises, mesurée dans une solution aqueuse à 4 % en poids et à une température de 20° C, avec un degré d'hydrolyse d'environ 85 % à 89 % et une concentration d'environ 0,1 % à 1 % en poids.

21. Procédé de production de microcapsules à libération prolongée selon la revendication 1, **caractérisé en ce que** l'excipient de lyophilisation est le mannitol à une concentration d'environ 0,1 à 5 % en poids par rapport à la suspension totale.

22. Procédé de production de microcapsules à libération prolongée selon la revendication 1, **caractérisé en ce que** les microcapsules dispersées dans une phase aqueuse et fractionnées dans les récipients pour le dosage final, sont congelées dans un congélateur à agitation orbitale jusqu'à des températures inférieures à -20° C pendant une durée d'environ 5 à 60 minutes.

23. Procédé de production de microcapsules à libération prolongée selon la revendication 1, **caractérisé en ce que** le congélateur à agitation orbitale utilisé pour la congélation est une plaque réfrigérée avec un mouvement orbital circulaire où le rayon de rotation est inférieur ou égal au rayon de la base des récipients adéquats, qui contiennent la dispersion aqueuse de microcapsules.

24. Procédé de production de microcapsules à libération prolongée selon la revendication 1, **caractérisé en ce que** le dispositif agitateur réalise un mouvement orbital circulaire, avec une vitesse de rotation d'environ 20 à 50 t/m.

25. Procédé de production de microcapsules à libération prolongée selon la revendication 1, **caractérisé en ce que** la centrifugation, le lavage, la dispersion des microcapsules dans un milieu aqueux et la congélation avec des opérations d'agitation sont effectués dans un système fermé.

26. Procédé de production de microcapsules à libération prolongée selon la revendication 1, **caractérisé en ce que**, pendant qu'il est effectué, les microcapsules conservent pratiquement la même taille, la même dispersion de diamètres de particules et la même concentration en peptide actif sont obtenues, pendant toute la durée pendant laquelle dure le processus.

27. Procédé de production de microcapsules à libération prolongée selon la revendication 1, **caractérisé en ce que** les flux d'alimentation des solutions ou dispersions qui entrent dans les équipements d'agitation intensive, sont ajustés de manière indépendante.

28. Procédé de production de microcapsules à libération prolongée selon la revendication 1, **caractérisé en ce que** les intensités d'agitation sont ajustées dans chaque équipement d'agitation intensive, indépendamment les unes des autres, et indépendamment des flux d'alimentation.

29. Procédé de production de microcapsules à libération prolongée selon la revendication 1, **caractérisé en ce que** le peptide actif est l'acétate de leuprolide.

30. Procédé de production de microcapsules à libération prolongée selon la revendication 1, **caractérisé en ce que** le peptide actif est l'acétate de goséréline.

31. Procédé de production de microcapsules à libération prolongée selon la revendication 1, **caractérisé en ce que** le principe actif est l'acétate de nafaréline.

32. Procédé de production de microcapsules à libération prolongée selon la revendication 1, **caractérisé en ce que** le peptide actif est l'acétate de triptoréline.

33. Procédé de production de microcapsules à libération prolongée selon la revendication 1, **caractérisé en ce que** le peptide actif est l'acétate de buséréline.

34. Procédé de production de microcapsules à libération prolongée selon la revendication 1, **caractérisé en ce que** le pourcentage de perte du peptide actif pendant l'ensemble du processus, par rapport à la quantité utilisée de matériau brut, est inférieur à 30 %.

35. Procédé de production de microcapsules à libération prolongée selon la revendication 1, **caractérisé en ce que** les récipients adéquats sont de petites fioles de dosage qui, après lyophilisation, sont fermées pour obtenir le produit final destiné à la consommation.

36. Procédé de production de microcapsules à libération prolongée selon la revendication 1, **caractérisé en ce que** les récipients adéquats sont des récipients qui permettent, après lyophilisation, d'obtenir le produit en vrac.

37. Procédé de production de microcapsules à libération prolongée selon la revendication 1, **caractérisé en ce que** la poudre des microcapsules résultant de ce procédé ne présente pas d'agglomération pendant un certain temps, et **en ce que** la reconstitution de la suspension à injecter est instantanée.
